# EUROPEAN PATENT SPECIFICATION

(11) **EP 2 349 289 B1**
(45) Date of publication and mention of the grant of the patent: **13.07.2016**
(21) Application number: 09824084.9
(22) Date of filing: 28.10.2009
(51) Int. Cl.: A61P 19/02, A61P 3/10, A61K 9/14, A61K 9/16, A61K 9/20, A61K 9/28, A61K 9/50, A61K 31/235, A61K 47/48

(54) **PHARMACEUTICAL COMPOSITIONS CONTAINING DIACEREIN**
PHARMAZEUTISCHE ZUSAMMENSETZUNGEN MIT DIACEREIN
COMPOSITIONS PHARMACEUTIQUES CONTENANT DE LA DIACÉRÉINE

(30) Priority: 28.10.2008 US 108931 P
(43) Date of publication of application: 03.08.2011
(73) Proprietor: TWI Biotechnology, Inc., Taipei 114 (TW)
(72) Inventor: GAO, Danchen, Long Grove, IL 60047 (US); WU, Jen-Sen, Wenshan District Taipei City 116 (TW); LU, Wei-Shu, Taipei County Tucheng City 236 (TW); CHEN, Shouchiung, East District Chiayi City 600 (TW); KUO, Pei-Chun, Songshan District Taipei City 105 (TW); CHEN, Chih-Ming, Xinyi District Tapei City 110 (TW)
(74) Representative: Kingsbury, Oliver William
(86) International application number: PCT/US2009/062302
(87) International publication number: WO 2010/051296

(56) References cited:
- EP-A1- 1 913 940
- WO-A2-2009/040702
- WO-A2-2009/048940
- US-A- 4 861 599
- US-A- 5 569 469
- US-A- 5 662 935
- US-A- 6 124 358
- US-A1- 2004 186 105
- US-A1- 2004 248 864
- US-A1- 2006 058 392
- US-A1- 2006 074 079
- US-A1- 2007 116 729
- US-B1- 6 197 818
- NICOLAS ET AL.: 'Clinical pharmacokinetics of diacerein.' CLIN PHARMACOKINET. vol. 5, 1998, pages 347 - 359, XP008147411

## Description

### BACKGROUND OF THE INVENTION

Diacerein (4, 5- bis(acetyloxy)-9, 10-dioxo-2-anthracene carboxylic acid) is a highly purified anthraquinone derivative. It is known to inhibit interleukin-1 activity, and has been approved as a Symptomatic Slow-Acting Drug in Osteoarthritis (SYSADOA) in several countries.

Diacerein has a log P value of 2.42 and is practically insoluble in water. Diacerein is entirely converted into rhein before reaching the systemic circulation. Rhein itself is either eliminated by the renal route (20%) or conjugated in the liver to rhein glucuronide (60%) and rhein sulfate (20%). These metabolites are mainly eliminated by the kidney.

There are two major adverse side effects of diacerein: diarrhea or soft stools and yellow-brown coloring of urine. The severity of diarrhea is mild-to-moderate and occurs within the first two weeks of treatment. Coloring of urine is due to the metabolites of diacerein present in the urine. *In vitro* and *in vivo* studies have showed that non- absorbed diacerein is metabolized to rhein in the colon. Rhein in the colon induces a laxative effect via activating chloride secretion by excitation of submucosal neurons and release of acetylcholine and endogenous prostaglandins, but not by release of histamine or serotonin.

Oral bioavailability of diacerein is about 35-56%. A 3-year clinical study indicated that up to 30% diarrhea or soft stools occurred in the patients who took diacerein twice a day with meals (M. Dougados et al., Arthritis & Rheumatism, 44(11), 2539-2547, 2001). Even though feeding increases the bioavailability of diacerein to 43∼70%, incomplete absorption still results in a local effect in the colon. The incidence rate of diarrhea was dose proportional, in contrast to a dose disproportional nature of the other side effects (J. P. Pelletier et al., Arthritis & Rheumatism, 43(10), 2339-2348, 2000). This finding implies that minimizing the exposure of diacerein to the colon could improve diarrhea symptoms by enhancing absorption in the intestine.

In addition to the treatment of osteoarthritis, diacerein may be considered for use in treating other inflammatory or autoimmune diseases, for example, type I/II diabetes and its complications, such as nephropathy, retinopathy, neuropathy or foot ulcers, etc. There are non-clinical studies indicating that diacerein and rhein slow down the disease progression of diabetes and suppress the hyper-metabolism of the kidney in diabetic animals. The potential mechanism of diacerein and its metabolite, rhein, to decrease the progression of type I/II diabetes and its complications involves decreasing the expression and activity of pro-inflammatory cytokines, IL-1; downregulating the expression of IL-6, TNF-α and TGF-β; and inhibiting iNOS expression; thereby decreasing the expression and function of GLUT-1 and decreasing the uptake of glucose.

US 2006/074079 relates to formulations in solid pharmaceutical forms containing diacereine and meloxicam. WO 2009/040702 relates to pharmaceutical compositions of rhein or diacerein, or salts or esters or prodrugs thereof. WO 2009/048940 relates to pharmaceutical formulations comprising diacerein or its derivatives having enhanced solubility properties of diacerein or its derivatives, and useful for administration. EP 1 913 940 relates to conjugates formed by the combination of rhein or their analogues with the organic bases or amino acids.

### SUMMARY OF THE INVENTION

An object of the present invention is to provide a once-daily controlled-release formulation of diacerein for treating inflammatory, autoimmune diseases or their complications, such as osteoarthritis, type I/II diabetes or diabetic nephropathy, with reduced adverse side effects. More specifically, the once-daily controlled-release formulations of diacerein of the present invention is a membrane-controlled formulation. In a preferred embodiment, the controlled-release formulations of diacerein of the present invention could further provide increased bioavailability when compared to commercial immediate release (IR) formulations. More specifically, said method reduces the adverse side effect of diarrhea caused by diacerein.

Yet another object of the invention is to provide a once-daily controlled-release formulation comprising diacerein and a second active ingredient for treating inflammatory, autoimmune diseases or their complications. More specifically, the second active ingredient could be an angiotensin converting enzyme inhibitor or an angiotensin II receptor blocker for treating diabetic nephropathy, an antihyperglycemic drug for treating type I/II diabetes, or a non-steroidal anti-inflammatory drug (NSAID) for treating osteoarthritis.

### DETAILED DESCRIPTION OF THE INVENTION

The major adverse side effects of diacerein are diarrhea and soft stools. *In vitro* and *in vivo* studies have showed that non-absorbed diacerein is metabolized to rhein in the colon. Rhein in the colon induces a laxative effect via activating chloride secretion by excitation of submucosal neurons and release of acetylcholine and endogenous prostaglandins, but not by release of histamine or serotonin.

The present invention provides a once-daily controlled-release formulation of diacerein which can minimize the release of diacerein in the colon to reduce these adverse side effects. An ideal control of diacerein release is when the drug release rate and the absorption rate are close to identical so that the adverse side effects caused by the contact of diacerein and the colon mucosa can be minimized. The technology used for controlling the release of diacerein is membrane-controlled technology.

The diacerein, or other active ingredient that is utilized in the present invention, may be prepared either through micronization alone or with a milling aid.

The diacerein utilized in the formulations of the present invention may be crystalline or in the amorphous state.

The sustained-release formulation may include common additives in addition to the active ingredient and a polymer. For example, the sustained-release core may include a diluent such as a microcrystalline cellulose, dextrose, starch, sucrose, lactose, sorbitol, mannitol or calcium phosphate; a disintegrating agent such as talc, sodium carboxymethylcellulose, L-hydroxypropylcellulose, cropovidone, or corn starch; a binder such as polyvinylpyrrolidone, starch, gelatin, tragacanth, methylcellulose, or hydroxypropylcellulose; and a solvent such as water or a lower alcohol such as ethanol or isopropanol; and a lubricant such as light anhydrous silicic acid, talc, stearic acid and its zinc, magnesium, or calcium salt or polyethyleneglycol. In addition, the sustained-release formulation may also include a disintegrating agent such as sodium starch glycolate, starch, alginic acid or its sodium salt.

A pharmaceutical composition of the present invention can be formulated as various types of oral formulations having the above-described composition. Preferably, the pharmaceutical composition of the present invention can be formulated as tablets or beads.

### Membrane-controlled Technology

The formulation of the invention may be surrounded by a controlled-release film that can isolate the drug core from the GI environment to minimize direct contact of diacerein with the colon mucosa.

The controlled-release film may contain a water-insoluble polymer which forms a membrane to avoid direct contact of diacerein and the colon mucosa. The water-insoluble polymer may include cellulose acetate, cellulose triacetate, agar acetate, amylose triacetate, beta glucan acetate, acetaldehyde dimethyl acetate, cellulose acetate methyl carbamate, cellulose acetate phthalate, cellulose acetate succinate, cellulose acetate dimethylamino acetate, cellulose acetate ethyl carbonate, cellulose acetate chloroacetate, cellulose acetate ethyl oxalate, cellulose acetate propionate, poly(vinylmethylether) copolymers, cellulose acetate butyl sulfonate, cellulose acetate octate, cellulose acetate laurate, cellulose acetate p-toluene sulfonate, triacetate of locust gum bean, hydroxylated ethylene-vinyl acetate, cellulose acetate butyrate, ethyl cellulose and the like.

The controlled-release film can further contain a plasticizer or a pore-forming agent to obtain suitable film properties. Examples of suitable plasticizers are dibutyl sebacate, triethyl citrate and polyethylene glycol (PEG). Examples of suitable pore-forming agents are hydroxymethylpropylcellulose (HPMC), polyvinylpyrrolidone (PVP) and hydroxypropylcellulose (HPC).

The drug release rate of diacerein can be controlled by adjusting the weight gain of the controlled-release film. Suitable weight gain could be 3-50% of the core tablet or bead.

The controlled-release formulation comprises an active layer, a sustained-release film layer and a delayed-release film layer.

In one embodiment of the invention, the active layer comprises between about 40.0% and about 50.0% by weight of microcrystalline cellulose, between about 20.0% and about 30.0% by weight of diacerein, between about 2.0% and about 5.0% by weight of povidone and between about 20.0% and about 30.0% by weight of mannitol.

In another embodiment of the invention, the active layer comprises about 50.0% by weight of microcrystalline cellulose, about 25.0% by weight of diacerein, about 2.0% by weight of povidone and about 23.0% by weight of mannitol.

The sustained-release film layer may comprise, but is not limited to, ethyl cellulose polymers, povidone, triethyl citrate and talc.

The delayed-release film layer may comprise, but is not limited to, Eudragit® polymers, triethyl citrate and talc.

### Methods for improving bioavailability

In another embodiment, the controlled-release formulation of the invention could further provide increased bioavailability of diacerein when compared to commercial immediate release formulations (ex. Arthrodar ®, TRB Pharma s.a.). It is believed that the increase in bioavailability could be helpful to decrease the adverse side effects. Methods for increasing the bioavailability include, but are not limited to, (a) adding surfactants; (b) forming a solid dispersion; (c) utilizing micronized or nanonized diacerein, (d) adding acidifying or buffering agents and (e) complexation with cyclodextrins.

The addition of suitable surfactants into pharmaceutical compositions of diacerein can enhance the *in vitro* dissolution rate and *in vivo* bioavailability. Suitable surfactants include, but are not limited to, sodium lauryl sulfate, polyethylene-polypropylene glycol, glycerol-polyethylene glycol oxystearate, PEG-40 hydrogenated castor oil and stearoyl macrogolglycerides (polyoxylglycerides).

Solid dispersions have been traditionally employed to enhance the dissolution rate of drugs, with a view to improve bioavailability. The drug may be entrapped in a carrier in an amorphous form without undergoing recrystallization. The process to prepare a solid dispersion is well known by a skilled artisan.

Controlling the particle size of diacerein is also considered to be helpful to improve its bioavailability. The preferred particle size of diacerein is D50 less than 20 µm and, more preferably, D50 less than 5 µm. In addition, the combination of co-micronized diacerein with hydrophilic milling aids can facilitate drug dissolution and bioavailability. Suitable hydrophilic milling aids include, but are not limited to, HPMC, sucrose, lactose, surfactants and superdisintegrants. The process may be practiced by utilizing a mill or a micronizer, such as an Aljet mill. The co-micronized diacerein can then be mixed or granulated with other excipients.

The tables below indicate the solubility and stability of diacerein in buffer solutions with different pH values. At a pH below 4.17, diacerein is stable and its solubility is relatively low. The degradation products including rhein increase at a pH above 5. The poor stability of diacerein in the intestinal environment may result in incomplete absorption and cause poor and variable bioavailability. Moreover, one of the increased degradants in the intestinal environment, rhein, has been suspected to be a major factor in stimulating colon mucosa and results in diarrhea. Accordingly, methods to stabilize diacerein during gastro-intestinal absorption might improve its bioavailability as well as the side effect of diarrhea. The stabilization methods for use
with diacerein may include the addition of acidifying or buffering agents or complexation with cyclodextrins.

**Table 1**

| Solubility and stability pH profile of diacerein at ambient temperature for 48 hours | | | | | | |
|---|---|---|---|---|---|---|
| **Buffer system** | **Final pH value** | **Total solubility (µg/mL)** | **Proportion of Diacerein and its degradants (%)** | | | |
| | | | **Diacerein** | **Rhein** | **Monoacetylrhein I** | **Monoacetylrhein II** |
| 0.1 N HCl | 1.17 | 0.17 | 100 | 0 | 0 | 0 |
| 0.01 N HCl | 1.98 | 0.15 | 100 | 0 | 0 | 0 |
| 50mM NaH₂PO₄ | 3.03 | 0.35 | 100 | 0 | 0 | 0 |
| | 4.17 | 0.43 | 100 | 0 | 0 | 0 |
| | 5.04 | 1.40 | 60 | 27 | 0 | 13 |
| | 5.97 | 152.7 | 77 | 10 | 8 | 5 |
| | 6.55 | 1105.2 | 45 | 28 | 14 | 13 |
| | 6.70 | 3746.2 | 34 | 36 | 16 | 14 |
| Water | 5.79 | 44.0 | 90 | 2 | 5 | 3 |

**Table 2**

| Solubility and stability of diacerein at ambient temperature for one hour | | | | | |
|---|---|---|---|---|---|
| **Final pH value** | **Total solubility (µg/mL)** | **Proportion of Diacerein and its degradants (%)** | | | |
| | | **Diacerein** | **Rhein** | **Monoacetylrhein I** | **Monoacetylrhein II** |
| *6.55** | *901.9* | *90* | *4* | *4* | *2* |
| *6.70** | *4444.1* | *83* | *4* | *7* | *6* |

### Use of the Formulations of the Invention

The pharmaceutical compositions of diacerein of the present invention are used for treating the inflammatory or autoimmune diseases rheumatoid arthritis, osteoarthritis, osteoporosis, inflammatory bowel disease, including ulcerative colitis and Crohn's disease, ulcerative colitis, multiple sclerosis, periodontitis, gingivitis, graft versus host reactions, psoriasis, scleroderma, atopic dermatitis, asthma, systemic lupus erythematosus (SLE), nephropathy and chronic obstructive pulmonary disease (COPD). Dermal conditions that are treated include those given above, and also psoriatic arthritis, epidermolysis bullosa, atopic dermatitis and vasculitis. Anti-angiogenic activity allows the treatment of conditions such as age-related macular degeneration and cancer. The pharmaceutical compositions of the invention are also used for treating osteoarthritis, type I/II diabetes or diabetic nephropathy, with fewer adverse side effects.

Suitable doses of diacerein for treating the above diseases are in the range of 5-200 mg/per day, preferably, 20-150 mg/per day.

When administered to a patient who has reached the steady state of plasma concentration, a 50 mg commercial IR diacerein formulation administered twice daily only maintains the plasma concentration of rhein above 2 mg/mL for about 12 hours. However, in a preferred embodiment of the invention:
a 50 mg diacerein formulation of the present invention maintains the plasma concentration of rhein above the concentration of 1 mg/mL for more than 12 hours in humans when orally administered to a human patient who has reached the steady state condition;
a 100 mg diacerein formulation of the present invention maintains the plasma concentration of rhein above the concentration of 2 mg/mL for more than 12 hours in humans when orally administered to a human patient who has reached the steady state condition;
a 150 mg diacerein formulation of the present invention maintains the plasma concentration of rhein above the concentration of 3 mg/L for more than 12 hours in humans when orally administered to a human patient who has reached the steady state condition;
and
a 200 mg diacerein formulation of the present invention maintains the plasma concentration of rhein above the concentration of 4 mg/L for more than 12 hours in humans when orally administered to a human patient who has reached the steady state condition.

### Combo formulations

The controlled-release formulation of the invention can further comprise another active ingredient, such as Angiotensin II receptor blockers (ARBs), angiotensin converting enzyme inhibitors (ACEIs), antihyperglycemics or NSAIDs. More specifically, the formulations of diacerein according to the present invention can further contain an angiotensin converting enzyme inhibitor or a angiotensin II receptor blocker for treating diabetic nephropathy, a antihyperglycemic drug for treating type I/II diabetes, or a non-steroidal anti-inflammatory drug (NSAID) for treating osteoarthritis.

Examples of ACEIs include captopril, benazepril, enalapril, lisinopril, fosinopril, ramipril, perindopril, quinapril, moexipril and trandolapril. Examples of ARBs include candesartan, eprosartan, irbesartan, telmisartan, valsartan and losartan. Examples of antihyperglycemics include sulfonylureas, such as glyburide, glipizide, and glimepiride; meglitinides such as repaglinide and nateglinide; biguanides such as metformin; thiazolidinediones such as pioglitazone and rosiglitazone; alpha glucosidase inhibitor such as acarbose. Examples of NSAIDs include salicylates such as aspirin; arylalkanoic acids, such as acetaminophen; 2-Arylpropionic acids such as Ibuprofen, Ketorolac and Naproxen; n-arylanthranilic acids such as mefenamic acid, meclofenamic acid; Oxicams such as piroxicam, meloxicam; and COX-2 inhibitors such as Celecoxib.

The second active ingredient may be in a controlled-release dosage form or in an immediate release dosage form.

It should be noted that the present embodiments are to be considered as illustrative and the invention is not to be limited to the details given herein.

### EXAMPLES

### Example 1 (not according to the invention)

### Preparation of solid dispersion

Acceptable ranges for the components of representative solid dispersions are shown in Table 3.

**Table 3**

| **Ingredients** | **Examples** | % |
|---|---|---|
| API | Diacerein | 10-90 |
| Carriers | hydrophilic polymers (HPMC, HPC, HEC, MC, sod. CMC etc.), hydrophobic polymers (EC, PVA, Methylmethacrylate etc.), | 10∼90 |
| | Surfactants (SLS, Tween 80, Gelucire, Cremophor, poloxamer, PEG etc.), water-soluble excipients (lactose, sucrose, mannitol, glucose etc.), | |
| | Wax (glyceryl behenate, cetyl alcohol etc.), or combinations thereof | |
| Organic solvents | Acetone, Isopropyl alcohol, or Ethanol | qs |

### Process:

Diacerein may be dissolved with suitable organic solvents to form a drug solution. Carriers, such as hydrophilic polymers, hydrophobic polymers, surfactants, water-soluble excipients, or wax, or a combination of the above carriers are then dissolved or dispersed in the drug solution. Spray drying of the above solution may be used to obtain a solid dispersion, or the solution may be coated onto suitable excipients (water-soluble materials that function as a second carrier) using a fluidized bed.

### Example 2 (not according to the invention)

### Complexation with cyclodextrins

Acceptable ranges for the components of representative complexes with cyclodextrins are shown in Table 4.

**Table 4**

| | Ingredients | % |
|---|---|---|
| API | Diacerein | 10∼90 |
| Cyclodextrins | α-cyclodextrin, β-cyclodextrin, γ-cyclodextrin and their derivatives, for example, 2-hydroxypropyl-β-cyclodextrin etc. | 10∼90 |

### Process:

Water solutions of cyclodextrins may be prepared with various percentages. Diacerein is added to the above solutions to yield saturated solutions. The solutions are stirred for at least 72 hours and then allowed to stand until all undissolved material has precipitated. The supernatant solution is filtered and dried by oven, spray drying or freeze drying or coated onto suitable excipients (which function as diluents) using a fluidized bed.

### Example 3 (not according to the invention)

### Matrix system (tablet)

Acceptable ranges for the components of representative tablet matrix systems are shown in Table 5.

**Table 5**

| | **Ingredients** | **%** |
|---|---|---|
| API | Diacerein, Diacerein in solid dispersion or in comlexation with cyclodextrins | 10∼90 |
| Controlled release materials | water-swellable polymers, hydrophilic polymers (HPMC, HPC, HEC, MC, sod. CMC etc.), hydrophobic polymers (EC, PVA, Methylmethacrylate etc.), wax (glyceryl behenate, cetyl alcohol etc.). | 10∼90 |

### Process:

The API part is prepared as described in the above examples. The diacerein API part is physically mixed or granulated with controlled release materials and then the mixture is compressed to obtain matrix tablets. Optionally, an acidifying agent or buffering agent may be included in the tablet formulation.

### Example 4 (not according to the invention)

### Matrix system (tablet)

Two representative matrix tablet formulations are shown in Table 6.

**Table 6**

| | | **Formula A** | | **Formula B** | |
|---|---|---|---|---|---|
| | **Ingredients** | mg | % | mg | % |
| **Granule I** | Diacerein | 100 | 20.0 | 100 | 20.0 |
| | HPC | 100 | 20.0 | -- | -- |
| | HPMC | -- | -- | 179 | 35.8 |
| | Mannitol | 204 | 40.8 | -- | -- |
| | SLS | 10 | 2.0 | -- | -- |
| | Cremophor | -- | -- | 10 | 2.0 |
| **Granule II** | HPMC | 80 | 16.0 | -- | -- |
| | Mannitol | -- | -- | 180 | 36.0 |
| | PVP | 3 | 0.6 | 3 | 0.6 |
| | Tartaric acid | | | 25 | 5.0 |
| **Lubricant** | Mg. stearate | 3 | 0.6 | -- | -- |
| | SiO2 | -- | -- | 3 | 0.6 |
| | Total | 500 | 100.0 | 500 | 100.0 |

### Process:

A solid dispersion of granule I was prepared as described in Example 1. Granule II was prepared by wet granulation. Granules I and II were mixed with lubricants and then compressed to obtain matrix tablets.

### Example 5

### (not according to the invention) Matrix system (tablet)

A further representative matrix tablet formulation is shown in Table 7.

**Table 7**

| | | **Formula C** | |
|---|---|---|---|
| | **Ingredients** | mg | % |
| **Granule** | Diacerein | 100 | 20.0 |
| | HPMC | 175 | 35.0 |
| | Mannitol | 147 | 29.4 |
| | Cremophor | 50 | 10.0 |
| | Tartaric acid | 25 | 5.0 |
| **Lubricant** | Mg. stearate | 3 | 0.6 |
| | **Total** | 500 | 100.0 |

### Process:

Diacerein, HPMC, mannitol, cremophor and tartaric acid were granulated by wet granulation. The granules were mixed with lubricants and then compressed.

### Example 6 (not according to the invention)

### Matrix system (beads)

Acceptable ranges for the components of representative bead matrix systems are shown in Table 8.

**Table 8**

| | **Ingredients** | **Percentage** |
|---|---|---|
| Core bead | | |
| API | Diacerein | 10∼90% of core |
| Carriers | hydrophilic polymers (HPMC, HPC, HEC, MC, sod. CMC etc.), | 10∼90% of core |
| | hydrophobic polymers (EC, PVA, Methylmethacrylate etc.), | |
| | Surfactants (SLS, Tween 80, Gelucire, Cremophor, poloxamer, PEG etc.), | |
| | water-soluble excipients (lactose, sucrose, mannitol, glucose etc.), | |
| | Wax (glyceryl behenate, cetyl alcohol etc.), or combinations thereof | |
| Seeds | Sphere of microcrystalline cellulose, sugar seed etc. | 10∼90% of core |

### Process:

Diacerein is dissolved with suitable organic solvents to form a drug solution. Carriers such as hydrophilic polymers, hydrophobic polymers, surfactants, water-soluble excipients, wax or the combination of above carriers are then dissolved or dispersed in the drug solution. The solution is sprayed onto seeds by fluidized bed to obtain matrix beads. The beads are then encapsulated in a capsule with suitable

### Example 7 (not according to the invention)

### Matrix system (beads)

A representative bead matrix formulation is shown in Table 9.

**Table 9**

| | | **Formula D** | |
|---|---|---|---|
| | **Ingredients** | mg | % |
| **Core Bead** | API | 100 | 20.0 |
| | HPC | 100 | 20.0 |
| | EC | 50 | 10.0 |
| | MCC sphere | 240 | 48.0 |
| | Cremophor | 10 | 2.0 |
| | **Total** | 500.0 | 100.0 |

The formula D was prepared by the process described in Example 6.

### Example 8

### Membrane-controlled system (tablet)

Acceptable ranges for the components of representative membrane controlled tablet formulations are shown in Table 10.

**Table 10**

| | ingredients | Percentage |
|---|---|---|
| Core tablet | | |
| API | Diacerein in solid dispersion or in complexation with cyclodextrins | 10∼90% of core |
| Diluents | Lactose, MCC, mannitol etc. | 10∼90% of core |
| Lubricants | Mg. stearate | 0.1∼5% of core |

| Film coating | | |
|---|---|---|
| Composition of controlled release membrane | hydrophobic polymers (EC, PVA, Methylmethacrylate etc.), pore-forming agent (HPMC, PVP, HPC, etc.) Plasticizer (dibutyl sebacate, Triethyl citrate, PEG etc.) | 3∼50% weight gain of core tablets |

The API part is prepared as described in the above examples. The Diacerein API part is physically mixed or granulated with suitable diluents and lubricants then compressed to obtain core tablets. Optionally, the acidifying agent or buffering agent may be included in the core tablet formulation. The controlled release materials are dissolved along with pore forming agents and plasticizer in organic solvents to obtain the coating solution for above core tablet. Then, the tablets are coated in a tablet coater.

### Example 9

### Membrane-controlled system (tablet)

Three representative membrane controlled tablet formulations are shown in Table 11.

**Table 11**

| | | **Formula E** | | **Formula F** | | **Formula G** | |
|---|---|---|---|---|---|---|---|
| | **Ingredients** | mg | % | mg | % | mg | % |
| **Core Tablet** | Diacerein | 100 | 22.7 | 100 | 23.0 | 100 | 23.0 |
| | Mannitol | 304 | 69.1 | 274.5 | 63.0 | 289.5 | 66.5 |
| | SLS | 20 | 4.5 | -- | -- | 30 | 6.9 |
| | Cremophor | -- | -- | 20 | 4.6 | -- | -- |
| | PVP | 14 | 3.2 | 14 | 3.2 | 14 | 3.2 |
| | Tartaric acid | -- | -- | 25 | 5.7 | -- | -- |
| | Mg. stearate | 2 | 0.5 | 2 | 0.5 | 2 | 0.5 |
| | *Subtotal* | 440 | 100 | 435.5 | 100 | 435.5 | 100 |
| **Seal Coat** | Core tablet | 440 | 97.3 | 435.5 | 97.3 | 435.5 | 97.3 |
| | HPMC | 8 | 1.8 | 8 | 1.8 | 8 | 1.8 |
| | Talc | 4 | 0.9 | 4 | 0.9 | 4 | 0.9 |
| | *Subtotal* | 452.0 | 100.0 | 447.5 | 100.0 | 447.5 | 100.0 |
| **SR Coat** | Seal-coated tablet | 452.0 | 90.4 | 447.5 | 89.5 | 447.5 | 89.5 |
| | EC | 30 | 6.0 | 25 | 5.0 | 25 | 5.0 |
| | PVP | 15 | 3.0 | 25 | 5.0 | 25 | 5.0 |
| | TEC | 3 | 0.6 | 2.5 | 0.5 | 2.5 | 0.5 |
| | **Total** | 500.0 | 100.0 | 500.0 | 100.0 | 500.0 | 100.0 |

### Process:

The core tablet was manufactured by a solid dispersion method as described in the above examples or by a wet granulation method. The core tablet was then coated with a seal coat and a sustained-release coat.

### Example 10

### Membrane-controlled system (beads)

Acceptable ranges for the components of representative bead membrane-controlled systems are shown in Table 12.

**Table 12**

| | **Ingredients** | **Percentage** |
|---|---|---|
| Core bead | | |
| API | Diacerein | 10∼90% of core |
| Carriers | hydrophilic polymers (HPMC, HPC, HEC, MC, sod. CMC etc.), | 10∼90% of core |
| | hydrophobic polymers (EC, PVA, Methylmethacrylate etc.), | |
| | Surfactants (SLS, Tween 80, Gelucire, Cremophor, poloxamer, PEG etc.), | |
| | water-soluble excipients (lactose, sucrose, mannitol, glucose etc.), | |
| | Wax (glyceryl behenate, cetyl alcohol etc.), or combinations thereof | |
| Seeds | Sphere of microcrystalline cellulose, sugar seed etc. | 10∼90% of core |

| Film coating | | |
|---|---|---|
| Composition of controlled release membrane | hydrophobic polymers (EC, PVA, Methylmethacrylate etc.), pore-forming agent (HPMC, PVP, HPC, etc.) Plasticizer (dibutyl sebacate, triethyl citrate, PEG etc.) | 3∼50% weight gain of core bead |

### Process:

Diacerein is dissolved with suitable organic solvents to form a drug solution. Carriers such as hydrophilic polymers, hydrophobic polymers, surfactants, water-soluble excipients, wax or the combination of above carriers are then dissolved or dispersed in the drug solution. The solution is sprayed onto seeds by a fluidized bed to obtain core beads. The controlled release materials are dissolved along with pore forming agents and plasticizer in organic solvents to obtain the coating solution for the core beads. Then, the beads are coated with a controlled-release membrane. The extended-release beads are then encapsulated in a capsule with suitable size.

### Example 11

### Membrane-controlled system (beads)

Representative bead membrane-controlled system formulations are shown in Table 13.

**Table 13**

| | | **Formula H** | |
|---|---|---|---|
| | **Ingredients** | mg | % |
| **Core Bead** | API | 100 | 22.8 |
| | HPC | 100 | 22.8 |
| | MCC sphere | 228.5 | 52.1 |
| | SLS | 10 | 2.3 |
| | *Subtotal* | 438.5 | 100 |
| **Seal Coat** | Core bead | 438.5 | 98.0 |
| | HPMC | 6 | 1.3 |
| | Talc | 3 | 0.7 |
| | *Subtotal* | 447.5 | 100.0 |
| **CR Coat** | Seal-coated bead | 447.5 | 89.5 |
| | EC | 25 | 5.0 |
| | PVP | 25 | 5.0 |
| | TEC | 2.5 | 0.5 |
| | **Total** | 500.0 | 100.0 |

The formula H was prepared by the process described in Example 10.

### Example 12 (not according to the invention)

### Osmotic pump (Push pull) system

Acceptable ranges for the components of representative osmotic pump (push pull) formulations are shown in Table 14.

**Table 14**

| | Ingredients | Example | Percentage |
|---|---|---|---|
| Drug layer | API | Diacerein, Diacerein in solid dispersion or in comlexation with cyclodextrins | 10∼90% of drug layer |
| | Osmotic agent | NaCl, mannitol, fructose etc. | 10∼90% of drug layer |
| | Osmotic polymer | Polyethylene oxide (PEO) | 10∼90% of drug layer |
| | Antioxidant | BHT | 0.01∼0.5% of PEO |
| | Binder | HPMC, PVP, HPC etc. | 0.5∼30% of drug layer |
| | Lubricant | Mg. stearate, SiO2 | |
| | | | 0.1∼5% of drug layer |
| Push layer | Osmotic agent | NaCl, mannitol, fructose etc. | 10∼90% of push layer |
| | Osmotic polymer | Polyethylene oxide (PEO) | |
| | Antioxidant | BHT | 10∼90% of push |
| | Binder | HPMC, PVP, HPC etc. | layer |
| | Lubricant | Mg. stearate, SiO2 | 0.01∼0.5% of PEO |
| | | | 0.5∼30% of push layer |
| | | | 0.1∼5% of push layer |
| Composition of semipermeable membrane | Film-former Pore-forming agent Plasticizer | Cellulose acetate (HPMC, PVP, HPC, etc.) dibutyl sebacate, triethyl citrate, PEG etc. | 3∼50% weight gain of bi-layered tablets |

The API part as described in the above examples is prepared by physically mixing or granulating the diacerein API part with PEO, an osmotic agent, a binder, and an antioxidant and then blending with a lubricant to obtain the drug layer. Optionally, the acidifying agent or buffering agent may be included in the drug layer formulation. The push layer is also prepared by physically mixing or granulating. The semipermeable membrane is introduced by dissolving cellulose acetate along with a pore forming agent and plasticizer in organic solvents and then performing the coating process in a tablet coater. A passageway is formed by laser or mechanical drilling on the surface of the CA film next to the drug layer.

### Example 13 (not according to the invention)

### Osmotic pump (Push pull) system

A representative push pull osmotic pump formulation is shown in Table 15.

**Table 15**

| | | **Formula I** | |
|---|---|---|---|
| | **Ingredients** | mg | % |
| **Drug Layer** | Diacerein | 100.00 | 20.00 |
| | PEO (MW 200,000) | 282.25 | 56.45 |
| | NaCl | 100.00 | 20.00 |
| | HPMC E5 | 15.00 | 3.00 |
| | BHT (Butylated hydroxytoluene) | 0.25 | 0.05 |
| | Glyceryl monostearate | 2.50 | 0.50 |
| | *subtotal* | 500.00 | 100.00 |
| **Push Layer** | PEO (MW 7,000,000) | 194.00 | 77.48 |
| | NaCl | 50.00 | 19.97 |
| | HPMC E5 | 5.00 | 2.00 |
| | BHT | 0.125 | 0.05 |
| | Glyceryl monostearate | 1.25 | 0.50 |
| | *subtotal* | 250.38 | 100 |
| **Semipermeable Membrane** | Bi-layer tablet | 750.38 | 83.34 |
| | Cellulose acetate (CA-398) | 112.5 | 12.49 |
| | PEG 4000 | 7.5 | 0.83 |
| | HPC (Klucel EF) | 30 | 3.33 |
| | Acetone / water* | qs | qs |
| | **Total** | 900.38 | 100.00 |

| | | | |
|---|---|---|---|
| *Which are evaporated during processing. | | | |

The formula I was prepared by the process described in Example 12.

### Example 14 (not according to the invention)

### Osmotic pump system (in-situ hole)

Acceptable ranges for the components of representative osmotic pump(in-situ hole) formulations are shown in **Table** 16.

**Table 16**

| | **Ingredients** | **Example** | **Percentage** |
|---|---|---|---|
| Core tablet | API | Diacerein, Diacerein in solid dispersion or in comlexation with cyclodextrins | 10∼90% of core tablet |
| | Osmotic agent | NaCl, mannitol, fructose etc. | 10∼90% of core |
| | Osmotic polymer | Polyethylene oxide (PEO) | tablet |
| | Antioxidant | BHT | 10∼90% of core |
| | Binder | HPMC, PVP, HPC etc. | tablet |
| | Lubricant | Mg. stearate, glyceryl monostearate, SiO2 etc. | 0.01∼0.5% of PEO |
| | | | 0.5∼30% of core tablet |
| | | | 0.1∼5% of core tablet |
| Composition of seal coat | Hydrophilic polymer Osmotic agent Lubricant | HPMC, HPC, PVP, HEC etc.(NaCl, sugars etc. (Talc, SiO2 etc.) | 0.5∼15% weight gain of core tablets |
| Composition of semipermeabl e membrane | Film-former Pore-forming agent Plasticizer | Cellulose acetate (HPMC, PVP, HPC, sugars etc. dibutyl sebacate, triethyl citrate, PEG etc. | 1∼20% weight gain of seal-coated tablets |

### Process:

The API part is prepared as described in the above examples. The diacerein API part is physically mixed or granulated with PEO, a binder, an osmotic agent and an antioxidant. The mixture is blended with lubricants and then compressed to obtain the core tablet. Optionally, the acidifying agent or buffering agent may be included in the core tablet formulation. A seal coating solution is prepared by dissolving or dispersing a hydrophilic polymer, an osmotic agent and lubricants in water, then spraying the coating solution onto the core tablets in a coater. A semipermeable coating is prepared by dissolving cellulose acetate along with a pore forming agent and plasticizer in an organic solvent and then spraying the coating solution onto the seal-coated tablet in a coater. At least one passageway is formed during the dissolution of the dosage form.

### Example 15 (not according to the invention)

### Osmotic pump system (in-situ hole)

A representative osmotic pump (in-situ hole) formulation is shown in Table 17.

**Table 17**

| | | **Formula J** | |
|---|---|---|---|
| | **Ingredients** | mg | % |
| **Drug Layer** | Diacerein | 100.00 | 19.93 |
| | PEO (MW 5,000,000) | 48.00 | 9.57 |
| | PEO (MW 200,000) | 192.00 | 38.27 |
| | NaCl | 139.00 | 27.70 |
| | Sodium lauryl sulfate | 15.00 | 2.99 |
| | BHT (Butylated hydroxytoluene) | 0.25 | 0.05 |
| | SiO2 | 2.50 | 0.50 |
| | Glyceryl monostearate | 5.00 | 1.00 |
| | *subtotal* | 501.75 | 100.00 |
| **Seal Coat** | Core tablet | 501.75 | 97.29 |
| | Opadry | 10.50 | 2.04 |
| | NaCl | 3.50 | 0.67 |
| | Water* | qs | qs |
| | *subtotal* | 515.75 | 100.00 |
| **Semipermeable Membrane** | Seal-coated tablet | 515.75 | 97.06 |
| | Cellulose acetate (CA-398) | 9.30 | 1.75 |
| | PEG 400 | 0.80 | 0.15 |
| | Triacetin | 0.80 | 0.15 |
| | Mannitol | 4.70 | 0.88 |
| | Acetone / water* | qs | qs |
| | **Total** | 531.35 | 100.00 |

| | | | |
|---|---|---|---|
| *Evaporated during processing. | | | |

The formula J was prepared by the process described in Example 14.

### Example 16 (not according to the invention)

### Sustained-release formulation (Matrix System)

Acceptable ranges for the components of representative matrix sustained release formulations are shown in Table 18.

**Table 18**

| | **Ingredients** | **Percentage** |
|---|---|---|
| Sustained Release Core | | |
| API | Diacerein, Micronized Diacerein or Comilled Diacerein, | 10∼90% of core |
| Sustained Release Polymer | water-swellable polymers, hydrophilic polymers (HPMC, HPC, HEC, MC, sod. CMC etc.), hydrophobic polymers (EC, PVA, Methylmethacrylate etc.), wax (glyceryl behenate, cetyl alcohol etc.). or combination of the polymers | 10-50% of core |
| Diluents | microcrystalline cellulose, dextrose, starch, sucrose, lactose, sorbitol, mannitol, and calcium phosphate | 10∼50% of core |
| Binder | PVP, HMPC, HPC | 1-20% of core |
| Disintegrant | L-HPC, sodium glycolate, croscarmellose sodium, | 1-10% of core |
| Lubricants | Mg. stearate | 0.1∼5% of core |

### Process:

The sustained-release formulation of the present invention can be prepared by direct compression, compaction-granulation, wet granulation or extrusion and spheronization.

In the case of using direct compression or compaction-granulation, the sustained-release formulation can be prepared in such a manner that the diacerein, a swellable polymer, a diluent, a disintegrating agent, a binder, and a lubricant are mixed, followed by granulation with a compaction granulator (e.g. roller compacter), screening through an about 20-mesh screen, and tabletting.

In the case of wet granulation, the sustained-release formulation can be prepared in such a manner that the diacerein, a swellable polymer, a diluent, a disintegrating agent, and a binder are mixed in a high shear granulator with the addition of water or solvent (e.g. ethanol or isopropyl alcohol). The granules are further dried, milled and mixed with lubricant and tabletting.

In the case of using extrusion and sphernoization, the sustained-release formulation can be prepared in such a manner that the diacerein, a swellable polymer, a diluent, a disintegrating agent, a binder, and a lubricant are mixed in a low shear granulator or mixer with the addition of water or solvent (e.g. ethanol or isopropyl alcohol). The wet mass is added to a single screw or twin screw extruder, the extrudate is spheronized in a marumerizer to obtain sustained release beads.

### Example 17 (not according to the invention)

A representative matrix sustained release tablet formulation is shown in Table 17.

**Table 19**

| | | **Formula K** | |
|---|---|---|---|
| | **Ingredients** | mg | % |
| **Granule** | Diacerein | 100 | 20.0 |
| | HPMC | 175 | 35.0 |
| | MCC | 147 | 29.4 |
| | SLS | 50 | 10.0 |
| | L-HPC | 25 | 5.0 |
| **Lubricant** | Mg. stearate | 3 | 0.6 |
| | **Total** | 500 | 100.0 |

Formula K was prepared by the process described in Example 16.

### Example 18

### Membrane Controlled System

Acceptable ranges for the components of representative sustained release membrane-controlled system formulations are shown in Table 20.

**Table 20**

| | **Ingredients** | **Percentage** |
|---|---|---|
| Core tablet or bead | | |
| API | Diacerein, micronized Diacerein or comilled Diacerein | 10∼90% of core |
| Diluents | Lactose, MCC, mannitol, sorbitol, etc. | 10∼90% of core |
| Binder | PVP, HPC, HPMC, etc | 1-10% of core |
| Lubricants | Mg. stearate | 0.1∼5% of core |
| Film coating | | |
| Composition of controlled release membrane | hydrophobic polymers (EC, PVA, Methylmethacrylate etc.), pore-forming agent (HPMC, PVP, HPC, etc.) Plasticizer (dibutyl sebacate, triethyl citrate, PEG etc.) | 3∼50% weight gain of core tablets |

### Process:

The core tablet is prepared by direct compression, compaction-granulation is used, or wet granulation. The core bead is prepared by fluid bed granulation.

When direct compression or compaction-granulation is used, the tablet core can be prepared in such a manner that the diacerein, a diluent, a binder, and a lubricant are mixed, followed by granulation with a compaction granulator (e.g. roller compacter), screening through an about 20-mesh screen, and tabletting.

When wet granulation is used, the core tablet can be prepared in such a manner that the diacerein, a diluent, and a binder are mixed in a high shear granulator with the addition of water or solvent (e.g. ethanol or isopropyl alcohol). The granules are further dried, milled and blended with lubricant and tabletted.

For bead core preparation, the beads can be prepared in such a manner that the diacerein, a diluent, and a binder are granulated in a fluid bed granulator with the addition of water or solvent (e.g. ethanol or isopropyl alcohol). The bead granules are further dried and sieved through an appropriate mesh.

The controlled release materials are dissolved along with pore forming agents and plasticizer in organic solvents to obtain the coating solution for the above core tablets or beads. Then, the tablets or beads are coated either in a tablet coater or a fluid bed coater.

### Example 19

### Bead formulation for a membrane-controlled system

A representative bead formulation for a membrane-controlled system is shown in Table 21.

**Table 21**

| | | **Formula L** | |
|---|---|---|---|
| | **Ingredients** | mg | % |
| **Core bead** | Diacerein | 100 | 22.7 |
| | Lactose | 302.6 | 68.8 |
| | SLS | 13.2 | 3 |
| | PVP | 22 | 5.0 |
| | Mg. stearate | 2.2 | 0.5 |
| | *Subtotal* | 440 | 100 |
| **Seal Coat** | Core beads | 440 | 97.3 |
| | HPMC | 8 | 1.8 |
| | Talc | 4 | 0.9 |
| **SR Coat** | *Subtotal* | 452.0 | 100.0 |
| | Seal-coated beads | 452.0 | 90.4 |
| | EC | 30 | 6.0 |
| | PVP | 15 | 3.0 |
| | TEC | 3 | 0.6 |
| | **Total** | 500.0 | 100.0 |

### Example 20 (not according to the invention)

### Hydrogel Matrix Formulations

Representative hydrogel matrix formulations are shown in Table 22.

**Table 22**

| | **DIAC-2001** | | **DIAC-2002** | | **DIAC-2006** | | **DIAC-2006** | | **DIAC-2017** | | **DIAC-2018** | |
|---|---|---|---|---|---|---|---|---|---|---|---|---|
| Ingredients | Mg/tab | % Solid | Mg/tab | % Solid | Mg/tab | % Solid | Mg/tab | % Solid | Mg/tab | % Solid | Mg/tab | % Solid |
| Diacerein (Highsun) | | | | | | | | | 50.0 | 20.0 | 50.0 | 20.0 |
| Diacerein (TRB) | 50.0 | 20.0 | 50.0 | 20.0 | | | | | | | | |
| Diacerein (Micronized) | | | | | 50.0 | 20.0 | 50.0 | 20.0 | | | | |
| HPMC K4MCR | 50.0 | 20.0 | 100.0 | 40.0% | 25.0 | 10.0 | | | | | | |
| HPMC K100LVCR | | | | | 25.0 | 10.0 | 50.0 | 20.0 | 75.0 | 30.0 | 100.0 | 40.0 |
| Mannitol | 142.5 | 57.0 | 92.5 | 37.0 | 142.5 | 57.0 | 142.5 | 57.0 | 115.0 | 46.0 | 90.0 | 36.0 |
| HPMC E5LV | | | | | | | | | 7.5 | 3.0 | 7.5 | 3.0 |
| Povidone K30 | 5.0 | 2.0 | 5.0 | 2.0 | 5.0 | 2.0 | 5.0 | 2.0 | | | | |
| Mg Stearate | 2.5 | 1.0 | 2.5 | 1.0 | 2.5 | 1.0 | 2.5 | 1.0 | 2.5 | 1.0 | 2.5 | 1.0 |
| **Total** | **250.0** | **100.0** | **250.0** | **100.0** | **250.0** | **100.0** | **250.0** | **100.0** | **250.0** | **100.0** | **250.0** | **100.0** |

Diacerein (Highsun) refers to diacerein produced by Taizhou Highsun Pharmaceutical Co., Ltd.

Diacerein (TRB) refers to diacerein produced by TRB Chemedica.
"HPMC K4MCR," "HPMC K100LVCR," and "HPMC E5LVCR" are various METHOCEL™ hypromellose products produced by the Dow Chemical Company.

### Example 21 (not according to the invention)

### Dissolution Data for Hydropel Matrix Formulations

Dissolution tests were performed on diacerein hydrogel matrix formulations of Example 20. The dissolution tests were performed according to the so-called "basket" method and/or the "paddle and sinker" method.

### "Basket" Method

The "basket method" uses USP apparatus 1. It is usually operated at 100 rpm (revolutions per minute) and is usually used for beads formulation. The FDA guidances contain descriptions of the "basket" method.

### "Paddle and Sinker Method"

The "paddle and sinker" method uses USP apparatus 2. It is usually operated at 50 rpm. A "sinker" can be some wires wrapped around the capsules before the capsules are put into dissolution vessels. The FDA guidances contain descriptions of the "paddle and sinker" method.

Both methods are usually used at 37° C ± 0.5° C. The samples are usually dissolved in 900 ml of aqueous media.

Table 23 contains the results of the dissolution tests performed on formulations DIAC-2002, DIAC-2005, DIAC-2017 and DIAC-2018. All tests were performed utilizing pH 6.0 PBS buffer. The tests on DIAC-2002 and DIAC-2005 formulations were performed utilizing the "basket" method at 100 rpm, and the tests on DIAC-2017 and DIAC-2018 formulations were performed utilizing the "paddle and sinker" method at 100 rpm.

**Table 23**

| | **DIAC-2002** | | **DIAC-2005** | | **DIAC-2017** | | **DIAC-2018** | |
|---|---|---|---|---|---|---|---|---|
| **Time (hr)** | **Mean** | **St. dev.** | **Mean** | **St. dev**. | **Mean** | **St. dev.** | **Mean** | **St. dev**. |
| 2 | 26 | 4.6 | 24 | 2.2 | 36 | 13.4 | 23 | 2.5 |
| 4 | 51 | 7.6 | 48 | 4.1 | 64 | 8.9 | 44 | 4.8 |
| 6 | 66 | 6.8 | 68 | 0.8 | 85 | 6.1 | 64 | 6.7 |
| 8 | 78 | 5.2 | 82 | 2.7 | 93 | 1.9 | 78 | 6.6 |
| 12 | 88 | 1.7 | 96 | 2.0 | 95 | 0.7 | 94 | 2.3 |
| 16 | 89 | 1.0 | 97 | 3.0 | 95 | 0.7 | 96 | 1.1 |

| | | | | | | | | |
|---|---|---|---|---|---|---|---|---|
| "St. dev." stands for "standard deviation." | | | | | | | | |

Table 24 contains the results of the dissolution tests performed on formulations DIAC-2001, DIAC-2002, DIAC-2005 and DIAC-2006. All tests were performed using pH 6.8 PBS buffer and the "basket" method at 100 rpm. The tests were performed in triplicates, and the table shows the data for the mean of these triplicates.

**Table 24**

| | **DIAC-2001** | | **DIAC-2002** | | **DlAC-2005** | | **DIAC-2006** | |
|---|---|---|---|---|---|---|---|---|
| Time(hr) | Mean | St.dev. | Mean | St.dev. | Mean | St.dev. | Mean | St.dev. |
| 2 | 23 | 0.2 | 12 | 0.3 | 32 | 9.0 | 49 | 3.6 |
| 4 | 44 | 0.1 | 22 | 1.4 | 54 | 14.9 | 78 | 7.0 |
| 6 | 58 | 0.7 | 31 | 3.6 | 69 | 16.8 | 93 | 3.8 |
| 8 | 68 | 1.2 | 39 | 5.0 | 77 | 13.9 | 90 | 4.0 |
| 12 | 79 | 1.4 | 50 | 5.0 | 85 | 4.3 | 86 | 3.8 |
| 16 | 87 | 1.1 | 59 | 7.0 | 86 | 0.5 | 83 | 3.8 |

### Example 22 (not according to the invention)

### Sustained-Release Formulations

Representative sustained release formulations are shown in Tables 25-29 as follows. Tables 25 and 26 show compositions of active layers of formulations DIAC-3002, DIAC-3004, DIAC-3006, DIAC-3007, DIAC-3008, DIAC-3010, DIAC-3011 and DIAC-3012; Tables 27 and 28 show compositions of Sustained-Release (SR) film layers of these formulations; and Table 29 shows compositions of Delayed-Release (DR) film layers of formulations DIAC-3007, DIAC-3008, DIAC-3011 and DIAC-3012 (the other formulations do not contain DR film layer).

**Table 25**

| **ACTIVE LAYER** | | | | | | | | |
|---|---|---|---|---|---|---|---|---|
| **Ingredients** | **DIAC-3002** | | **DIAC-3004** | | **DIAC-3006** | | **DIAC-3007** | |
| | mg/cap | %Solid | mg/cap | %Solid | mg/cap | %Solid | mg/cap | %Solid |
| Cellets® 350 | 100 | 48.8% | | | 100.0 | 47.6% | 100.0 | 47.6% |
| Cellets® 700 | | | 100.0 | 47.6% | | | | |
| Diacerein (Highsun) | 50.0 | 24.4% | 50.0 | 23.8% | 50.0 | 23.8% | 50.0 | 23.8% |
| Diacerein (TRB) | | | | | | | | |
| Povidone K30 | 5.0 | 2.4% | 10.0 | 4.8 | 10.0 | 4.8% | 10.0 | 4.8% |
| Mannitol | 50.0 | 24.4% | 50.0 | 23.8% | 50.0 | 23.8% | 50.0 | 23.8% |
| **Subtotal** | **205.0** | **100.0%** | **210.0** | **100.0%** | **210.0** | **100.0%** | **210.0** | **100.0%** |

Cellets® 350 and Cellets® 750 are neutral starter cores for controlled release formulation produced by the Glatt Group.

**Table 26**

| **ACTIVE LAYER** | | | | | | | | |
|---|---|---|---|---|---|---|---|---|
| **Ingredients** | **DIAC-3008** | | **DIAC-3010** | | **DIAC-3011** | | **DIAC-3012** | |
| | mg/cap | %Solid | mg/cap | %Solid | mg/cap | %Solid | mg/cap %Solid | |
| Cellets® 350 | 100.0 | 47.6% | 100.0 | 47.6% | 100.0 | 47.6% | 100.0 | 47.6% |
| Diacerein (Highsun) | 50.0 | 23.8% | | | | | | |
| Diacerein (TRB) | | | 50.0 | 23.8% | 50.0 | 23.8% | 50.0 | 23.8% |
| Povidone K30 | 10.0 | 4.8% | 10.0 | 4.8% | 10.0 | 4.8% | 10.0 | 4.8% |
| Mannitol | 50.0 | 23.8% | 50.0 | 23.8% | 50.0 | 23.8% | 50.0 | 23.8% |
| **Subtotal** | **210.0** | **100.0%** | **210.0** | **100.0%** | **210.0** | **100.0%** | **210.0** | **100.0%** |

**Table 27**

| **SR FILM LAYER** | | | | | | | | |
|---|---|---|---|---|---|---|---|---|
| **Ingredients** | **DIAC-3002** | | **DIAC-3004** | | **DIAC-3006** | | **DIAC-3007** | |
| | mg/cap | %Solid | mg/cap | %Solid | mg/cap | %Solid | mg/cap | %Solid |
| Ethocel 10 cps | | 36.4% | | 36.4% | | 36.4% | 21.0 | 36.4% |
| Povidone K30 | | 36.4% | | 36.4% | | 36.4% | 21.0 | 36.4% |
| Triethyl Citrate | | 7.3% | | 7.3% | | 7.3% | 4.2 | 7.3% |
| Talc | | 20.0% | | 20.0% | | 20.0% | 11.5 | 20.0% |
| **Subtotal** | | **100.0%** | | **100.0%** | | **100.0%** | **57.6** | **100.0%** |

**Table 28**

| **SR FILM** | | | | | | | | |
|---|---|---|---|---|---|---|---|---|
| **Ingredients** | **DIAC-3008** | | **DIAC-3010** | | **DIAC-3011** | | **DIAC-3012** | |
| | mg/cap | %Solid | mg/cap | %Solid | mg/cap | %Solid | mg/cap | %Solid |
| Ethocel 10 cps | 21.0 | 36.4% | | 36.4% | 18.2 | 36.4% | 18.2 | 36.4% |
| Povidone K30 | 21.0 | 36.4% | | 36.4% | 18.2 | 36.4% | 18.2 | 36.4% |
| Triethyl Citrate | 4.2 | 7.3% | | 7.3% | 3.6 | 7.2% | 3.6 | 7.2% |
| Talc | 11.5 | 20.0% | | 20.0% | 10.0 | 20.0% | 10.0 | 20.0% |
| **Subtotal** | **57**.**6** | **100.0%** | | **100.0%** | 50.0 | **100.0%** | 50.0 | **100.0%** |

**Table 29**

| **DR FILM LAYER** | | | | |
|---|---|---|---|---|
| **Ingredients** | **DIAC-3007** | **DIAC-3008** | **DIAC-3011** | **DIAC-3012** |
| | % Solid | % Solid | % Solid | % Solid |
| Eudragit L30D-55 | 66.9% | | | |
| Eudragit S100 | | 66.9% | | 66.9% |
| Eudragit L100 | | | 66.9% | |
| Tiethyl Citrate | 10.0% | 10.0% | 10.0% | 10.0% |
| Talc | 23.1% | 23.1% | 23.1% | 23.1% |
| **Subtotal** | **100.0%** | **100.0%** | **100%** | **100%** |

### Example 23 (not according to the invention)

### Dissolution Data for Sustained-Release Formulations

Dissolution tests were performed on diacerein sustained-release formulations of Example 22. The dissolution tests were performed according to the "basket" method and/or the "paddle and sinker" method as described in Example 21.

Table 30 contains the results of the dissolution tests performed on formulations DIAC-3002, DIAC-3004, DIAC-3006 and DIAC-3007. All tests were performed utilizing pH 6.0 PBS buffer and utilizing the "basket" method at 100 rpm.

**Table 30**

| **Time(hr)** | **DIAC-3002** | | **DIAC-3004** | | **DIAC-3006** | | **DIAC-3007** | |
|---|---|---|---|---|---|---|---|---|
| | 6% SR* | 18% SR | 7% SR | 16% SR | 6% SR | 18% SR | 19% DR | |
| | Mean | Mean | Mean | Mean | Mean | Mean | Mean | |
| 2 | 26 | 13 | 9 | 7 | 29 | 16 | 23 | |
| 4 | 49 | 25 | 15 | 12 | 51 | 27 | 35 | |
| 6 | 69 | 35 | 21 | 16 | 72 | 38 | 45 | |
| 8 | 85 | 46 | 27 | 20 | 88 | 48 | 54 | |
| 12 | 99 | 64 | 39 | 28 | 102 | 67 | 72 | |
| 16 | 102 | 80 | 51 | 35 | 105 | 82 | 85 | |

| | | | | | | | | |
|---|---|---|---|---|---|---|---|---|
| *The % refers to the Sustained-Release (SR) or the Delayed-Release (DR) polymer percentage in each tested formulation. | | | | | | | | |

Table 31 contains the results of the dissolution tests performed on formulations DIAC-3010 and DIAC-3011. All tests were performed utilizing pH 6.0 PBS buffer and utilizing the "basket" method at 100 rpm.

**Table 31**

| **Time(hr)** | **DIAC-3010** | **DIAC-3011** | |
|---|---|---|---|
| | 16% SR | 6% DR | 19% DR |
| | Mean | Mean | Mean |
| 2 | 4 | 5 | 3 |
| 4 | 7 | 10 | 8 |
| 6 | 11 | 14 | 13 |
| 8 | 14 | 19 | 17 |
| 12 | 20 | 26 | 25 |
| 16 | 26 | 34 | 33 |

Table 32 contains the results of the dissolution tests performed on formulations DIAC-3004, DIAC-3006 and DIAC-3007. All tests were performed utilizing pH 6.8 PBS buffer and utilizing the "basket" method at 100 rpm.

**Table 32**

| **Time(hr)** | **DIAC-3004** | | **DIAC-3006** | **DIAC-3007** |
|---|---|---|---|---|
| | 7% SR | 16% SR | 18% SR | 19% DR |
| | Mean | Mean | Mean | Mean |
| 2 | 46 | 25 | 67 | 92 |
| 4 | 84 | 48 | 93 | 120 |
| 6 | 98 | 69 | 97 | 119 |
| 8 | 97 | 86 | 95 | 116 |
| 12 | 93 | 95 | 91 | 112 |
| 16 | 92 | 93 | 89 | 110 |

Table 33 contains the results of the dissolution tests performed on formulations DIAC-3008, DIAC-3010, DIAC-3011 and DIAC-3012. All tests were performed utilizing pH 6.8 PBS buffer and utilizing the "basket" method at 100 rpm.

**Table 33**

| **Time(hr)** | **DIAC-3008** | | **DIAC-3010** | | **DIAC-3011** | | **DIAC-3012** |
|---|---|---|---|---|---|---|---|
| | 5% DR | 13% DR | 7% SR | 16% SR | 7% DR | 19% DR | 5% DR |
| | Mean | Mean | Mean | Mean | Mean | Mean | Mean |
| 2 | 15 | 5 | 44 | 22 | 28 | 27 | 1 |
| 4 | 58 | 9 | 72 | 42 | 54 | 52 | 2 |
| 6 | 87 | 15 | 86 | 59 | 75 | 74 | 4 |
| 8 | 95 | 27 | 91 | 74 | 91 | 91 | 7 |
| 12 | 93 | 69 | 92 | 90 | 98 | 103 | 16 |
| 16 | 91 | 101 | 91 | 92 | 96 | 101 | 32 |

## Claims

1. A once-daily controlled-release formulation of diacerein for use in the treatment of inflammatory diseases, autoimmune diseases or their complications with reduced adverse side effects, selected from rheumatoid arthritis, osteoarthritis, osteoporosis, inflammatory bowel disease, Crohn's disease, ulcerative colitis, multiple sclerosis, periodontitis, gingivitis, graft versus host reactions, psoriasis, scleroderma, atopic dermatitis, asthma, systemic lupus erythematosus (SLE), nephropathy, chronic obstructive pulmonary disease (COPD), psoriatic arthritis, epidermolysis bullosa, atopic dermatitis, vasculitis, age-related macular degeneration, cancer, type I/II diabetes and diabetic nephropathy, the formulation comprising an active layer, a sustained-release film layer, and a delayed-release film layer, wherein the formulation is a membrane-controlled formulation.

2. The controlled-release formulation according to claim 1, wherein the formulation comprises a surfactant, acidifying agent or a buffering agent.

3. The controlled-release formulation according to claim 1, wherein the particle size of diacerein is less than 20 µm.

4. The controlled-release formulation according to claim 1, wherein the diacerein is presented as an amorphous state in a solid carrier.

5. The controlled-release formulation according to claim 1, wherein the diacerein is formed as a complex with cyclodextrins.

6. The controlled release formulation according to claim 1, wherein the diacerein is crystalline.

7. The controlled release formulation according to claim 1, wherein the controlled-release formulation is for use in treating type I/type II diabetes or osteoarthritis.

8. The controlled-release formulation according to claim 1, wherein said active layer comprises between 40.0% and 50.0% by weight of microcrystalline cellulose, between 20.0% and 30.0% by weight of diacerein, between 2.0% and 5.0% by weight of povidone and between 20.0% and 30.0% by weight of mannitol.

## Patentansprüche

1. Formulierung von Diacerin für einmal tägliche kontrollierte Freisetzung zur Verwendung bei der Behandlung von entzündlichen Krankheiten, Autoimmunkrankheiten oder ihren Komplikationen mit verringerten nachteiligen Nebenwirkungen, ausgewählt aus rheumatoider Arthritis, Osteoarthritis, Osteoporose, entzündlicher Darmerkrankung, Morbus Crohn, Colitis ulcerosa, multipler Sklerose, Periodontitis, Gingivitis, Graft-versus-Host-Reaktionen, Psoriasis, Scleroderma, atopischer Dermatitis, Asthma, systemischem Lupus erythematodes (SLE), Nephropathie, chronisch obstruktiver Lungenerkrankung (COPD), Psoriasisarthritis, Epidermolysis bullosa, atopischer Dermatitis, Vaskulitis, altersbedingter Makuladegeneration, Krebs, Typ-I-/Typ-II-Diabetes und diabetischer Nephropathie, wobei die Formulierung eine aktive Schicht, eine Filmschicht für zeitgesteuerte Freisetzung und eine Filmschicht für verzögerte Freigabe umfasst, wobei die Formulierung eine membrangesteuerte Formulierung ist.

2. Formulierung für kontrollierte Freisetzung nach Anspruch 1, wobei die Formulierung ein Tensid, Säuerungsmittel oder ein Puffermittel umfasst.

3. Formulierung für kontrollierte Freisetzung nach Anspruch 1, wobei die Partikelgröße von Diacerin weniger als 20 µm beträgt.

4. Formulierung für kontrollierte Freisetzung nach Anspruch 1, wobei das Diacerin als ein amorpher Zustand in einem festen Träger vorliegt.

5. Formulierung für kontrollierte Freisetzung nach Anspruch 1, wobei das Diacerin als ein Komplex mit Cyclodextrinen gebildet ist.

6. Formulierung für kontrollierte Freisetzung nach Anspruch 1, wobei das Diacerin kristallin ist.

7. Formulierung für kontrollierte Freisetzung nach Anspruch 1, wobei die Formulierung für kontrollierte Freisetzung zur Verwendung bei der Behandlung von Typ-I-/Typ-II-Diabetes oder Osteoarthritis dient.

8. Formulierung für kontrollierte Freisetzung nach Anspruch 1, wobei die aktive Schicht zwischen 40,0 und 50,0 Gewichtsprozent mikrokristalline Cellulose, zwischen 20,0 und 30, Gewichtsprozent Diacerin, zwischen 2,0 und 5,0 Gewichtsprozent Povidon und zwischen 20,0 und 30,0 Gewichtsprozent Mannitol umfasst.

## Revendications

1. Formulation à libération contrôlée, à prendre une fois par jour, de diacéréine pour utilisation dans le traitement de maladies inflammatoires, de maladies auto-immunes ou leurs complications avec réduction des effets secondaires indésirables, sélectionnées parmi la polyarthrite rhumatoïde, l'arthrose, l'ostéoporose, la maladie intestinale inflammatoire, la maladie de Crohn, la colite ulcéreuse, la sclérose en plaques, la parodontite, la gingivite, les réactions du greffon contre l'hôte, la sclérodermie, le psoriasis, la dermatite atopique, l'asthme, le lupus érythémateux disséminé (LED), la néphropathie, la maladie pulmonaire obstructive chronique (MPOC), l'arthrite psoriasique, l'épidermolyse bulleuse, la dermatite atopique, la vascularite, la dégénérescence maculaire liée à l'âge, le cancer, le diabète de type I/II et la néphropathie diabétique, la formulation comportant une couche active, une couche de film à libération prolongée et une couche de film à libération retardée, où la formulation est une formulation contrôlée par membrane.

2. Formulation à libération contrôlée selon la revendication 1, où la formulation comprend un tensioactif, un agent acidifiant ou un agent de tamponnage.

3. Formulation à libération contrôlée selon la revendication 1, où la taille des particules de diacéréine est inférieure à 20 µm.

4. Formulation à libération contrôlée selon la revendication 1, où la diacéréine se présente à l'état amorphe dans un véhicule solide.

5. Formulation à libération contrôlée selon la revendication 1, où la diacéréine est sous forme de complexe avec les cyclodextrines.

6. Formulation à libération contrôlée selon la revendication 1, où la diacéréine est cristalline.

7. Formulation à libération contrôlée selon la revendication 1, où la formulation à libération contrôlée est utilisée dans le traitement du diabète de type I/Type II ou de l'arthrose.

8. Formulation à libération contrôlée selon la revendication 1, où ladite couche active comprend entre 40,0% et 50,0% en poids de cellulose microcristalline, entre 20,0% et 30,0% en poids de diacéréine, entre 2,0% et 5,0% en poids de povidone et entre 20,0% et 30,0% en poids de mannitol.
